(19) Europäisches Patentamt
European Patent Office
Office européen des brevets

(11) EP 0 733 700 A1

(12) **DEMANDE DE BREVET EUROPEEN**

(43) Date de publication:
**25.09.1996 Bulletin 1996/39**

(51) Int Cl.$^6$: **C11D 3/37**, C11D 17/00, A61K 7/06, A61K 7/00

(21) Numéro de dépôt: **96420079.4**

(22) Date de dépôt: **12.03.1996**

(84) Etats contractants désignés:
**BE DE GB**

(30) Priorité: **23.03.1995 FR 9503646**

(71) Demandeur: **COATEX S.A.**
**F-69730 Genay (FR)**

(72) Inventeurs:
- **Kensicher, Yves**
  **69380 Lozanne (FR)**
- **Suau, Jean-Marc**
  **69480 Lucenay (FR)**

(54) **Utilisation d'agents amphotères comme modificateurs de phases lamellaires de compositions détergentes ou cosmétiques liquides ou pâteuses**

(57) Utilisation de copolymères amphotères de formule générale :

$$(X)_a\text{-}(Y)_b\text{-}(Z)_c$$

dans laquelle

- X représente le motif monomérique à charge anionique
- Y représente le motif monomérique à charge non ionique
- Z représente le motif monomérique à charge cationique

avec a, pourcentage en poids, par rapport à la masse totale des monomères compriss entre 95 et 15
avec b, pourcentage en poids, par rapport à la masse totale des monomères compris entre 0 et 65
avec c, pourcentage en poids, par rapport à la masse totale des monomères compris entre 5 et 60
comme agent modificateur de rhéologie de phases lamellaires de compositions détergentes ou cosmétiques liquides ou pâteuses.

Lessive liquide et shampooing liquide contenant le dit agent.

EP 0 733 700 A1

## Description

L'invention a pour objet l'application de copolymères comprenant au moins deux motifs monomériques dont le premier est composé d'un ou plusieurs monomères éthyléniques à charge anionique, le deuxième est composé d'un ou plusieurs monomères éthyléniques à charge cationique dont l'un au moins de ces monomères à charge cationique possède une structure tensio-active, et comprenant éventuellement un troisième motif qui est composé d'un ou plusieurs monomères éthyléniques à caractère non ionique, comme agent amphotère modificateur de rhéologie de phases lamellaires en phase aqueuse.

L'invention a également pour objet l'application des dits copolymères modificateurs de rhéologie de phases lamellaires à l'épaississement ou à la fluidification de compositions détergentes ou cosmétiques, liquides ou pâteuses, quelle que soit l'ionicité des ingrédients constituant les dites compositions c'est-à-dire anionique, cationique ou non-ionique ou bien encore l'application à la compatibilité du mélange d'anionique et de cationique.

L'invention concerne également les compositions détergentes ou cosmétiques, liquides ou pâteuses, contenant le dit agent.

De nos jours pour des raisons de facilité d'emploi, les consommateurs utilisent de plus en plus de compositions détergentes liquides. Ces compositions pour être efficaces contiennent des tensio-actifs anioniques et non ioniques qui forment des micelles en phase aqueuse qui s'arrangent sous forme de lamelles plus ou moins concentrées lorsque des "builders" du type borax, citrate, formate de sodium ou bien encore des sels d'acide faible sont incorporés en plus ou moins grande quantité dans la composition.

Il est alors communément dit qu'il s'agit de phase lamellaire anionique. De même lorsque ces compositions contiennent des tensio-actifs cationiques et non ioniques, l'utilisation de plus ou moins grandes quantités de ces mêmes "builders" aboutit à la formation de phases lamellaires cationiques plus ou moins concentrées.

Or la présence de ces phases lamellaires qu'elles soient anioniques ou cationiques conditionne la viscosité et la stabilité de la composition détergente liquide tout comme la présence simultanée de phases lamellaires anioniques et cationiques lorsque par exemple il est souhaité de proposer aux consommateurs un seul produit commercial comportant une composition détergente liquide anionique associée à un adoucissant ou assouplissant cationique ou bien encore par exemple une composition shampooing anionique associée à un conditionneur de cheveux cationique ou amphotère.

Si ces phases lamellaires sont trop fortement concentrées, elles occupent alors une forte fraction volumique du liquide occasionnant ainsi une forte élévation de la viscosité de la formulation liquide ainsi qu'une floculation de ces micelles pouvant aboutir à la non coulabilité de la composition.

A l'inverse si ces phases lamellaires sont trop faiblement concentrées, elles ont alors tendance à sédimenter et à occasionner une perte de stabilité de la composition. Ce même type de problème se rencontre également dans les formulations cosmétiques du type shampooing.

Jusqu'à présent l'homme du métier a proposé différentes solutions pour résoudre ce problème de viscosité et de stabilité de la composition finale.

Ainsi la demande de brevet EP 0 346 995 propose l'utilisation d'un polymère défloculant comprenant un squelette hydrophile et une ou plusieurs chaînes latérales hydrophobes mais cette solution ne permet pas d'obtenir la compatibilisation de phases lamellaires anioniques avec des phases lamellaires cationiques lorsque celles-ci sont présentes conjointement dans la formulation.

De même le brevet US 5 275 809 révèle l'utilisation de terpolymère à base de chlorure de diméthyldiallyle ammonium dans des formulations anioniques de shampooing. Mais là encore, ce type de copolymère ne permet pas la compatibilité avec n'importe quelle formulation anionique ou cationique.

Une autre solution a été envisagée dans la demande EP 0 564 250 en proposant l'utilisation de peroxyacides ayant une solubilité à l'eau particulière.

Il a maintenant été découvert que l'utilisation comme modificateur de rhéologie de phases lamellaires en phase aqueuse de polymère amphotère, comprenant au moins deux motifs monomériques dont le premier est constitué d'un ou plusieurs monomères éthyléniques à charge anionique, le deuxième est constitué d'un ou plusieurs monomères éthyléniques à charge cationique, dont l'un au moins de ces monomères à charge cationique possède une structure tensio-active c'est-à-dire constituée d'une ou plusieurs chaînes latérales composées de groupements alkyle, aryle, alkylaryle ou arylalkyle possédant au moins 8 atomes de carbone et comprenant éventuellement un troisième motif constitué d'un ou plusieurs motifs à caractère non ionique, permet de manière surprenante, par les interactions ioniques entre le dit polymère et les différentes phases lamellaires en phase aqueuse, d'atteindre l'équilibre entre la stabilité et la viscosité des compositions liquides ou pâteuses, détergentes ou cosmétiques telles que par exemple les shampooings ainsi que la compatibilité des diverses phases lamellaires anioniques et cationiques lorsque les compositions liquides ou pâteuses comprennent à la fois des phases anioniques et cationiques.

Ainsi un des buts de l'invention est l'utilisation, pour modifier la rhéologie de phases lamellaires en phase aqueuse de compositions détergentes ou cosmétiques liquides ou pâteuses comme les shampooings, de copolymères répondant à la formule générale :

$$(X)_a - (Y)_b - (Z)_c$$

dans laquelle

- X représente le motif monomérique à charge anionique constitué d'un ou plusieurs monomères éthyléniques à charge anionique
- Y représente le motif monomérique à caractère non ionique constitué d'un ou plusieurs monomères à charge non ionique
- Z représente le motif monomérique à charge cationique constitué d'un ou plusieurs monomères éthyléniques à charge cationique dont l'un au moins de ces monomères possède une structure tensio-active c'est-à-dire constituée d'une ou plusieurs chaînes latérales composées de groupements alkyle, aryle, alkylaryle ou arylalkyle possédant au moins 8 atomes de carbone et dans laquelle

  a représentant le pourcentage en poids par rapport à la masse totale des monomères est compris, bornes incluses, entre 95 et 15,
  b représentant le pourcentage en poids par rapport à la masse totale des monomères est compris, bornes incluses, entre 0 et 65,
  c représentant le pourcentage en poids par rapport à la masse totale des monomères est compris, bornes incluses, entre 5 et 60, et plus particulièrement entre 10 et 35.

Ainsi un autre but de l'invention est l'application des dits copolymères à l'épaississement ou à la fluidification de compositions détergentes ou cosmétiques liquides ou pâteuses ou bien encore à la compatibilité des diverses phases des compositions détergentes ou cosmétiques.

De plus, un autre but de l'invention est de fournir un agent de modification de rhéologie des phases lamellaires composé du dit copolymère.

Enfin un but supplémentaire de l'invention est de fournir des compositions détergentes et cosmétiques, liquides ou pâteuses, telles que par exemple les shampooings comprenant le dit agent modificateur de rhéologie de phases lamellaires en phase aqueuse.

Ainsi alors que l'art antérieur décrit pour l'essentiel l'utilisation de polymères défloculants comprenant un squelette hydrophile et une ou plusieurs chaînes latérales hydrophobes, l'utilisation selon l'invention s'en distingue par le fait que l'agent modificateur de rhéologie selon l'invention se compose toujours d'au moins deux motifs monomériques dont le premier est constitué d'un ou plusieurs monomères éthyléniques à charge anionique, le deuxième est constitué d'un ou plusieurs monomères éthyléniques à charge cationique dont l'un au moins de ces monomères à charge cationique possède une structure tensio-active c'est-à-dire constituée d'une ou plusieurs chaînes latérales composées de groupements alkyle, aryle, alkylaryle ou arylalkyle possédant au moins 8 atomes de carbone et comprenant éventuellement un troisième motif constitué d'un ou plusieurs motifs à caractère non ionique, et répond à la formule générale (I) :

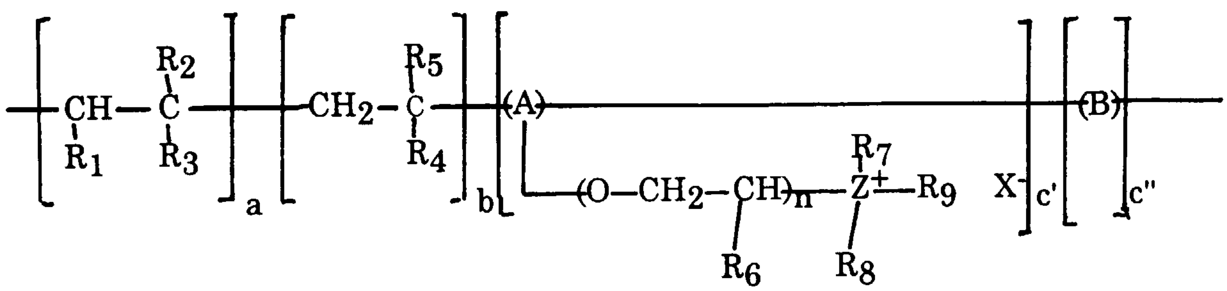

dans laquelle :

- Pour le motif de type anionique

  $R_1$ est H ou COOH
  $R_2$ est H ou $CH_3$
  $R_3$ est un groupement comportant au moins une fonction acide éventuellement totalement ou partiellement salifiée,

et a représentant le pourcentage en poids par rapport à la masse totale en monomère, est compris, bornes incluses, entre 95 et 15.

- Pour le motif de type non ionique

$R_4$ est -CO-$NH_2$, -CO-$OR_4$', -CO-$NR_4$"$R_4$"',

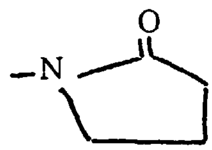

dans lequel
$R_4$' est un radical alkyle ou oxyalkyle ayant 1 à 4 atomes de carbone,
$R_4$" est H ou un radical alkyle ayant 1 à 4 atomes de carbone,
$R_4$"' est un radical alkyle ayant 1 à 4 atomes de carbone,
puis
$R_5$ est H ou $CH_3$

et b représentant le pourcentage en poids par rapport à la masse totale en monomère, est compris, bornes incluses, entre 0 et 65.

Pour le motif de type cationique,

- A représente le radical insaturé polymérisable et polymérisé, appartenant au groupe des esters acrylique, méthacrylique, maléique, itaconique, crotonique, vinylphtalique ainsi que les hémiesters maléique, itaconique, vinylphtalique ou encore les insaturés uréthannes tels que par exemple les acryluréthanne, méthacryluréthanne, α-α, diméthyl- m- isopropenylbenzyluréthanne, allyluréthanne ou bien encore les éthers allyliques, les acrylamide et méthacrylamide substituées ou non, des vinyliques.
- $R_6$ est H ou $CH_3$
- n=2 à 30
- $R_7$ est un alkyle ayant 1 à 4 atomes de carbone
- Z est N ou S

X est un contre-ion sulfate ou halogènure et lorsque Z est N :

$R_8$ est une chaîne alkyle ayant 8 à 22 atomes de carbone ou un motif de formule :

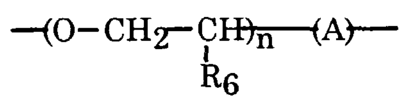

avec n = 2 à 30

et

$R_9$ est une chaîne alkyle ayant 8 à 22 atomes de carbone,

et lorsque Z est S :

$R_8$ n'existe pas
$R_9$ est une chaîne alkyle ayant 8 à 22 atomes de carbone.

et c' représentant le pourcentage en poids, par rapport à la masse totale en monomère, du monomère à charge cationique possédant une structure tensio-active, est compris, bornes incluses, entre 5 et 60 et préférentiellement entre 10 et 35 avec c'+c" = c , c représentant le pourcentage en poids de tous les monomères cationiques et variant entre 5 et 60 et préférentiellement entre 10 et 35.

- B représente un motif monomérique cationique choisi parmi les monomères suivants : l'acrylate et/ou le méthacrylate de triméthyl amino éthyl chlorure et/ou sulfate, l'acrylamide et/ou le méthacrylamido de triméthylamino propyl chorure et/ou sulfate

et c" représentant le pourcentage en poids, par rapport à la masse totale en monomère, du monomère cationique ne possédant pas la structure tensio-active, est compris, bornes incluses, entre 0 et 55 avec c' + c" = c, c représentant le pourcentage en poids de tous les monomères à charge cationique et variant entre 5 et 60, et plus particulièrement entre 10 et 35.

Ces copolymères résultent, selon les procédés connus de la synthèse ou de la copolymérisation radicalaire en solution, en émulsion directe ou inverse, en suspension ou précipitation, en présence d'initiateurs et des régulateurs appropriés, en milieux aqueux, alcoolique, hydroalcoolique, aromatique, aliphatique ou dans un solvant halogéné d'au moins deux motifs monomèriques définis ci dessus.

Ainsi, le milieu de copolymérisation peut être l'eau, le méthanol, le propanol, l'isopropanol, les butanols, ou encore le diméthylformamide, le diméthylsulfoxyde, le tétrahydrofurane, l'acétone, le méthyléthylcétone, l'acétate d'éthyle, l'acétate de butyle, l'hexane, l'heptane, le benzène, le toluène, l'éthylbenzène, le xylène, les solvants halogénés comme le tétrachlorure de carbone, le chloroforme, le dichlorométhane, les éthers de monopropylèneglycol, diéthylèneglycol.

Les copolymères destinés à être utilisés dans l'application, selon l'invention, à la fluidification de compositions détergentes liquides ou de compositions cosmétiques liquides ou pâteuses sont généralement choisis parmi ceux ayant une viscosité spécifique comprise entre 0,3 et 3,0 et préférentiellement entre 0,4 et 2,0.

D'autre part les copolymères destinés à être utilisés dans l'application, selon l'invention, à l'épaississement de compositions détergentes liquides ou de compositions cosmétiques liquides ou pâteuses sont généralement choisis parmi ceux ayant une viscosité spécifique supérieure à 3,0 et préférentiellement supérieure à 10.

La viscosité spécifique des copolymères sélectionnés, qui est symbolisée par le lettre "$\eta$" est déterminée de la manière suivante :

On prépare une solution de copolymère sous forme de sel sodique par dissolution de 50 g sec du copolymère dans un litre d'une solution d'eau distillée contenant 60 g de chlorure de sodium.

Puis, on mesure avec un viscosimètre capillaire placé dans un bain thermostaté à 25°C le temps d'écoulement d'un volume donné de la solution précitée contenant le copolymère alcalin, ainsi que le temps d'écoulement du même volume de solution aqueuse de chlorure de sodium dépourvue dudit copolymère. Il est alors possible de définir la viscosité spécifique "$\eta$" grâce à la relation suivante :

$$\eta=\frac{\text{(Temps d'écoulement de la solution de copolymère) - (Temps d'écoulement de la solution de NaCl)}}{\text{(Temps d'écoulement de la solution NaCl)}}$$

Le tube capillaire est généralement choisi de telle manière que le temps d'écoulement de la solution de NaCl dépourvue de copolymère soit d'environ 90 à 100 secondes, donnant ainsi des mesures de viscosité spécifique d'une très bonne précision.

Dès la fin de la polymérisation, les copolymères acides en solution aqueuse sont recueillis et peuvent selon l'invention être mis en oeuvre sous cette forme.

Puis les copolymères sélectionnés en solution aqueuse sont totalement ou partiellement neutralisés par un agent de neutralisation disposant d'une fonction monovalente tel que les cations alcalins.

En pratique, la phase liquide ou hétérogène résultant de la copolymérisation et contenant le copolymère acide sélectionné peut être utilisée sous une forme salifiée comme modificateur de rhéologie mais elle peut également être séchée par tous les moyens connus pour en éliminer cette phase et isoler le copolymère sous la forme d'une fine poudre et être utilisée sous cette autre forme.

Dans une plus large perspective, l'agent modificateur de rhéologie utilisé dans l'application selon l'invention peut être mis en oeuvre selon la méthode de préparation d'une composition détergente ou cosmétique, liquide ou pâteuse, la dite méthode comprenant les étapes de réalisation suivante :

a) on réalise, sous agitation rapide en évitant la formation de trop de bulles, la préparation de la phase lamellaire anionique ou cationique par introduction, suivant la nature de la phase lamellaire, de tensio-actifs anioniques, cationiques et/ou non ioniques dans de l'eau éventuellement alcalinisée suivi d'un ajout éventuel d'autres additifs tels que par exemple des builders, du propylène glycol, des azurants optiques, des colorants et autres.
b) on introduit en continu dans la phase lamellaire ainsi préparée, au moins 0,25 % en poids sec par rapport au poids total de la phase lamellaire d'agent modificateur de rhéologie de phases lamellaires en phase aqueuse
c) et dans le cas de la fabrication d'un mélange stable et homogène de phases cationiques et anioniques, on procède au mélange des phases préparées selon l'étape a) et on rajoute au moins 0,25 % en poids sec, par rapport au poids total du mélange, d'agent amphotère modificateur de rhéologie de phases lamellaires en phase aqueuse.

D'une manière générale les tensio-actifs anioniques utilisés dans la préparation de la phase lamellaire anionique sont tels que par exemple les alkyl-benzène sulfonates parmi lesquels le tétrapropylène benzène sulfonate, les alcane-sulfonates, les alkylsulfates, les alkyléthersulfates et autres.

De même dans la préparation des phases lamellaires cationiques les tensio-actifs cationiques sont entre autres et par exemple des sels d'imidazoline, des chlorures de dialkyldiméthylammonium, des chlorures d'alkyldiméthylbenzyl

ammonium.

De plus pour tous les types de formulations les tensio-actifs non ioniques sont choisis par exemple parmi les éthoxylats d'alcools gras, d'alcools oxo, d'alkylphénols ainsi que les éthers d'alkyl polyglycol, d'alkylphénol polyglycol ou bien encore les alcanolamides d'acide gras ou autres.

De même les divers additifs rentrant dans la préparation des phases lamellaires sont par exemple des builders, du propylène glycol, des azurants optiques, des colorants ou autres.

Dès lors que la quantité de l'agent modificateur de rhéologie de phases lamellaires en phase aqueuse introduite dans la phase lamellaire permet d'aboutir à la fluidification ou à la viscosification et à la stabilité souhaitée, on obtient la composition détergente ou cosmétique, liquide ou pâteuse, selon l'invention contenant le dit modificateur de rhéologie de phases lamellaires en phase aqueuse.

La portée et l'intérêt de l'invention seront mieux perçus grâce aux exemples suivants qui ne sauraient présenter un caractère limitatif.

EXEMPLE 1:

Cet exemple concerne la fluidification d'une composition détergente liquide anionique par l'utilisation d'agent amphotère modificateur de rhéologie de phases lamellaires, selon l'invention.

Dans ce but, pour chacun des essais de l'exemple, on prépare la composition de lessive liquide anionique en introduisant dans un bécher de 5 litres muni d'une pale d'agitation de 70 millimètres de diamètre, 1400 g d'eau et 10,6 g de soude perle. Après dissolution complète de la soude on intoduit sous agitation et successivement 25,7 g de mono éthanolamine, 257,3 g de dodécylbenzène sulfonate de sodium et 695,9 g de nonylphénol oxyéthylé 9 fois avant d'introduire en dernier lieu et simultanément 30,5 g de carbonate de soude et 144,1 g de citrate trisodique.

On laisse alors l'agitation se poursuivre pendant 5 minutes avant de rajouter la quantité suffisante d'agent amphotère correspondant à 0,56 % en poids sec d'agent amphotère par rapport au poids total de la formulation.

Après 20 minutes d'agitation de la composition ainsi réalisée, on mesure les viscosités des différents essais à 10 tours/minute, 20 tours/minute et 100 tours/minute à 25°C et à l'aide d'un viscosimètre Brookfield type RVT équipé du mobile adéquat. Les différents essais effectués mettent en oeuvre les agents amphotères modificateurs de rhéologie de phases lamellaires suivants :

Essai n°1 :

Cet essai est l'essai témoin dans lequel aucun agent amphotère n'est introduit.

Essai n°2 :

Cet essai illustre l'invention et met en oeuvre un agent amphotère modificateur de rhéologie de phases lamellaires dont la composition monomérique est :

47,4 % en poids d'acide acrylique pour le monomère à charge anionique

41,8 % en poids d'acrylamide pour le monomère à caractère non ionique et

10,8 % en poids d'un monomère à charge cationique de formule :

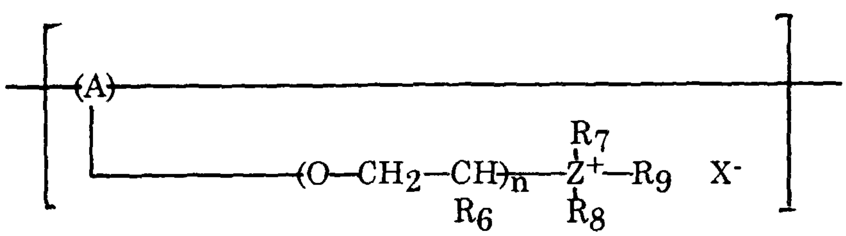

dans laquelle

(A) est un radical insaturé polymérisable et polymérisé, produit de la réaction de condensation du méthacrylate d'éthylène glycol avec le toluène diisocyanate

$R_6$ est H ,

n = 5-m

$R_7$ est $CH_3$,

$R_8$ représente le radical -(A)-(O-$CH_2$-$CH_2$-$)_m$ avec m + n = 5

$R_9$ est un radical alkyle à 12 atomes de carbone

Z = N

$X = SO_4CH_3$
et dont la viscosité spécifique est égale à 0,56.

Essai n° 3 :

Cet essai illustre l'invention et met en oeuvre un agent amphotère modificateur de rhéologie de phases lamellaires dont la composition monomérique est identique à celle de l'agent de l'essai n° 2 mais dont la viscosité spécifique est égale à 0,80.

Essai n° 4 :

Cet essai illustre l'invention et met en oeuvre un agent amphotère modificateur de rhéologie de phases lamellaires dont la composition monomérique est identique à celle de l'agent de l'essai n° 2 mais dont la viscosité spécifique est égale à 2,03.

Essai n° 5 :

Cet essai illustre l'invention et met en oeuvre un agent amphotère modificateur de rhéologie de phases lamellaires dont la composition monomérique est :

- 41,9 % en poids d'un mélange équimolaire d'acide acrylique et d'acide méthacrylique pour le monomère à charge anionique
- 41,0 % en poids d'acrylamide pour le monomère à caractère non ionique
- 10,6 % en poids de monomère à charge cationique de même formule que celui de l'essai n° 2 à charge cationique
- 6,5 % en poids de méthacrylate de triméthylaminoéthyl chlorure d'ammonium comme deuxième monomère à charge cationique, et dont la viscosité spécifique est égale à 0,67.

Essai n° 6 :

Cet essai illustre l'invention et met en oeuvre un agent amphotère modificateur de rhéologie de phases lamellaires dont la composition monomérique est :

- 36,6 % en poids d'acide acrylique pour le monomère à charge anionique
- 40,2 % en poids d'acrylamide pour le monomère à caractère non ionique
- 10,4 % en poids de monomère à charge cationique de même formule que celui de l'essai n° 2 à charge cationique
- 12,8 % en poids de méthacrylate de triméthylaminoéthyl chlorure d'ammonium comme deuxième monomère à charge cationique, et dont la viscosité spécifique est égale à 0,56.

Essai n° 7 :

Cet essai illustre l'invention et met en oeuvre un agent amphotère modificateur de rhéologie de phases lamellaires dont la composition monomérique est :

- 47,4 % en poids d'acide acrylique pour le monomère à charge anionique
- 10,8 % en poids de monomère à charge cationique de même formule que celui de l'essai n° 2, à charge cationique
- 41,8 % en poids de n-vinyl pyrrolidone pour le monomère à caractère non ionique,

et dont la viscosité spécifique est égale à 0,78.

Essai n°8 :

Cet essai illustre l'invention et met en oeuvre un agent amphotère modificateur de rhéologie de phases lamellaires dont la composition monomérique est :

- 89,2 % en poids d'acide acrylique pour le monomère à charge anionique
- 10,8 % en poids de monomère à charge cationique de même formule que celui de l'essai n° 2 à charge cationique,

et dont la viscosité spécifique est égale à 0,46.

Essai n°9 :

Cet essai illustre l'invention et met en oeuvre un agent amphotère modificateur de rhéologie de phases lamellaires dont la composition monomérique est :

- 18,1 % en poids d'acide acrylique pour le monomère à charge anionique
- 59,8 % en poids d'acrylamide pour le monomère à caractère non ionique
- 22,1 % en poids d'un monomère à charge cationique de formule :

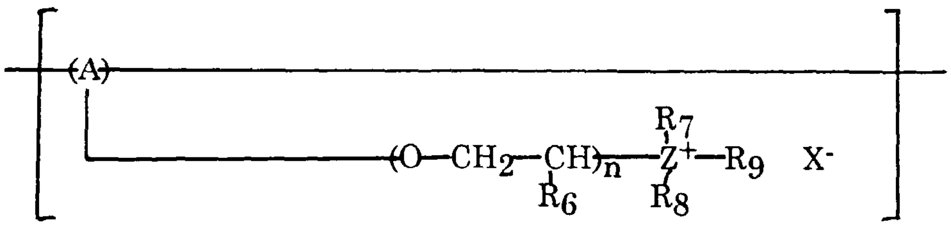

dans laquelle

(A) représente le radical méthacrylate
$R_6$ est H
n = 15 - m
$R_7 = CH_3$
$R_8$ est $(A)\text{-}(O\text{-}CH_2\text{-}CH_2\text{-})_m$ avec m + n = 15
Z est N
$R_9$ est un radical alkyle à 12 atomes de carbone
X est Cl

et dont la viscosité spécifique est égale à 0,80.

Les résultats des mesures de viscosité Brookfield des divers essais sont rassemblés dans le tableau 1 ci-après, sachant que pour tous les essais les compositions obtenues, selon l'invention, sont stables et homogènes.

## TABLEAU 1

| | Essai n° | MODIFICATEUR DE RHEOLOGIE | | | | FORMULATION DETERGENTE | | | |
|---|---|---|---|---|---|---|---|---|---|
| | | Compositions monomères | | | Viscosité Spécifique | Quantité modificateur (% en poids) | VISCOSITE BROOKFIELD ( mPa.s) | | |
| | | Anionique (% en poids) | Non ionique (% en poids) | Cationique (% en poids) | | | 10 T/min | 20 T/min | 100 T/min |
| TEMOIN | 1 | - | - | - | - | - | 5400 | 4600 | 3400 |
| INVENTION | 2 | 47,4 | 41,8 | 10,8 | 0,56 | 0,56 | 4800 | 4300 | 3300 |
| | 3 | 47,4 | 41,8 | 10,8 | 0,80 | 0,56 | 3900 | 1800 | 690 |
| | 4 | 47,4 | 41,8 | 10,8 | 2,03 | 0,56 | 2200 | 1500 | 620 |
| | 5 | 41,9 | 41,0 | 17,1 | 0,67 | 0,56 | 3000 | 1800 | 680 |
| | 6 | 36,6 | 40,2 | 23,2 | 0,56 | 0,56 | 1900 | 1100 | 370 |
| | 7 | 47,4 | 41,8 | 10,8 | 0,78 | 0,56 | 4500 | 2600 | 890 |
| | 8 | 89,2 | 0 | 10,8 | 0,46 | 0,56 | 1000 | 750 | 380 |
| | 9 | 18,1 | 59,8 | 22,1 | 0,80 | 0,56 | 1100 | 800 | 400 |

La lecture du tableau 1 permet de constater la fluidification c'est-à-dire la baisse de viscosité Brookfield à 10 tours/minute, 20 tours/minute et 100 tours/minute de la composition détergente liquide anionique témoin lorsqu'on utilise, selon l'invention, un agent amphotère modificateur de rhéologie de phases lamellaires de viscosité spécifique comprise entre 0,3 et 3,0 et plus particulièrement entre 0,4 et 2,0.

EXEMPLE 2 :

Cet exemple concerne la fluidification d'une composition détergente liquide cationique par l'utilisation, selon l'invention, d'agent amphotère modificateur de rhéologie de phases lamellaires.

Dans ce but, pour chacun des essais de l'exemple, on prépare la composition de lessive liquide cationique en introduisant sous agitation dans un bécher de 5 litres muni d'une pale d'agitation de 70 millimètres de diamètre 1352 g d'eau et successivement 780 g de nonylphénol oxyéthylé 9 fois, 130 g d'ammonium quaternaire commercialisé par la société CECA sous le nom NORAMIUM M2SH1 et 338 g de citrate trisodique.

On laisse alors l'agitation se poursuivre pendant 5 minutes avant de rajouter la quantité suffisante d'agent amphotère correspondant à 0.56 % en poids sec d'agent amphotère par rapport au poids total de la formulation. Après 20 minutes d'agitation de la composition ainsi réalisée, on mesure les viscosités des différents essais à 10 tours/minute, 20 tours/minute et 100 tours/minute à 25°C et à l'aide d'un viscosimètre Brookfield type RVT équipé du mobile adéquat.

Les différents essais effectués mettent en oeuvre les agents amphotères modificateurs de rhéologie de phases lamellaires suivants:

Essai n° 10 :

Cet essai est l'essai témoin dans lequel aucun agent amphotère modificateur de rhéologie de phases lamellaires n'est introduit.

Essai n° 11 :

Cet essai illustre l'invention et met en oeuvre le même agent amphotère modificateur de rhéologie de phases lamellaires que celui de l'essai n° 3.

Essai n° 12 :

Cet essai illustre l'invention et met en oeuvre le même agent modificateur de rhéologie de phases lamellaires que celui de l'essai n° 4.

Essai n° 13 :

Cet essai illustre l'invention et met en oeuvre un agent amphotère modificateur de rhéologie de phases lamellaires dont la composition monomérique est identique à celle des essais n° 11 et n° 12 mais dont la viscosité spécifique est égale à 0,59.

Essai n° 14 :

Cet essai illustre l'invention et met en oeuvre le même agent amphotère modificateur de rhéologie de phases lamellaires que celui de l'essai n° 5.

Essai n° 15 :

Cet essai illustre l'invention et met en oeuvre le même agent amphotère modificateur de rhéologie de phases lamellaires que celui de l'essai n° 6.

Essai n° 16 :

Cet essai illustre l'invention et met en oeuvre un agent amphotère modificateur de rhéologie de phases lamellaires, dont la composition monomérique est :

- 26,4 % en poids d'acide acrylique pour le monomère à charge anionique
- 38,7 % en poids d'acrylamide pour le monomère à caractère non ionique
- 10,0 % en poids de monomère à charge cationique de même formule que celui de l'essai n° 2 à charge cationique

- 24,9 % en poids de méthacrylate de triméthylaminoéthyl chlorure d'ammonium comme deuxième monomère à charge cationique,

et dont la viscosité spécifique est égale à 0,44.

Essai n° 17 :

Cet essai illustre l'invention et met en oeuvre un agent amphotère modificateur de rhéologie de phases lamellaires dont la composition monomérique est :

- 15,4 % en poids d'acide acrylique pour le monomère à charge anionique
- 32,1 % en poids d'acrylamide pour le monomère à caractère non ionique
- 8,3 % en poids de monomère à charge cationique de même formule que celui de l'essai n° 2 à charge cationique
- 44,2 % en poids de méthacrylate de triméthylamino éthyl chlorure d'ammonium comme deuxième monomère à charge cationique,

et dont la viscosité spécifique est égale à 0,36.

Essai n° 18 :

Cet essai illustre l'invention et met en oeuvre le même agent amphotère modificateur de rhéologie de phases lamellaires que celui de l'essai n°8.

Essai n° 19 :

Cet essai illustre l'invention et met en oeuvre le même agent amphotère modificateur de rhéologie de phases lamellaires que celui de l'essai n° 9.

Les résultats des mesures de viscosité Brookfield des divers essais sont rassemblés dans le tableau 2 ci-après, sachant que pour tous les essais les compositions obtenues, selon l'invention, sont stables et homogènes.

## TABLEAU 2

| | Essai n° | MODIFICATEUR DE RHEOLOGIE – Composition monomères – Anionique (% en poids) | Non ionique (% en poids) | Cationique (% en poids) | Viscosité Spécifique | FORMULATION DETERGENTE – Quantité (% en poids) | VISCOSITE BROOKFIELD (mPa.s) – 10 T/min | 20 T/min | 100 T/min |
|---|---|---|---|---|---|---|---|---|---|
| TEMOIN | 10 | - | - | - | - | - | 14400 | 6700 | 1440 |
| INVENTION | 11 | 47,4 | 41,8 | 10,8 | 0,80 | 0,56 | 140 | 140 | 125 |
| INVENTION | 12 | 47,4 | 41,8 | 10,8 | 2,03 | 0,56 | 100 | 100 | 95 |
| INVENTION | 13 | 47,4 | 41,8 | 10,8 | 0,59 | 0,56 | 560 | 460 | 182 |
| INVENTION | 14 | 41,9 | 41,0 | 17,1 | 0,67 | 0,56 | 160 | 160 | 116 |
| INVENTION | 15 | 36,6 | 40,2 | 23,2 | 0,56 | 0,56 | 1200 | 820 | 345 |
| INVENTION | 16 | 26,4 | 38,7 | 34,9 | 0,44 | 0,56 | 1000 | 600 | 130 |
| INVENTION | 17 | 15,4 | 32,1 | 52,5 | 0,36 | 0,56 | 1200 | 820 | 230 |
| INVENTION | 18 | 89,2 | 0 | 10,8 | 0,46 | 0,56 | 1280 | 890 | 500 |
| INVENTION | 19 | 18,1 | 59,8 | 22,1 | 0,80 | 0,56 | 110 | 104 | 104 |

La lecture du tableau 2 permet de constater la fluidification c'est-à-dire la baisse de viscosité Brookfield à 10 tours/minute, 20 tours/minute et 100 tours/minute de la composition détergente liquide cationique témoin lorsqu'on utilise, selon l'invention, un agent amphotère modificateur de rhéologie de phases lamellaires de viscosité spécifique comprise

entre 0,3 et 3,0 et plus particulièrement entre 0,4 et 2,0.

EXEMPLE 3 :

Cet exemple concerne l'épaississement d'une composition détergente liquide anionique par l'utilisation, selon l'invention, d'agent amphotère modificateur de rhéologie de phases lamellaires.

Dans ce but, pour chacun des essais de l'exemple, on prépare la composition de lessive liquide anionique en introduisant sous agitation dans un bécher de 5 litres muni d'une pale d'agitation de 70 millimètres de diamètre 361,3 g d'eau et 2,6 g de soude perle. Après dissolution complète de la soude on introduit sous agitation et successivement 6,36 g de monoéthanolamine 63,6 g de dodécylbenzène sulfonate de sodium et 152,1 g de nonylphénol oxyéthylé 9 fois, avant d'introduire en dernier lieu et simultanément 7,5 g de carbonate de soude et 36,5 g de citrate trisodique.

On laisse alors l'agitation se poursuivre pendant 5 minutes avant de rajouter la quantité suffisante d'agent amphotère correspondant à 0.89 % en poids sec d'agent amphotère par rapport au poids total de la formulation. Après 20 minutes d'agitation de la composition ainsi réalisée, on mesure les viscosités des différents essais à 10 tours/minute, 20 tours/minute et 100 tours/minute à 25°C et à l'aide d'un viscosimètre Brookfield type RVT équipé du mobile adéquat. Les différents essais effectués mettent en oeuvre les agents amphotères modificateur de rhéologie de phases lamellaires suivants:

Essai n° 20 :

Cet essai est l'essai témoin dans lequel aucun agent amphotère n'est introduit.

Essai n° 21 :

Cet essai illustre l'invention et met en oeuvre un agent amphotère modificateur de rhéologie de phases lamellaires dont la composition monomérique est :

- 47,4 % en poids d'acide acrylique pour le monomère à charge anionique
- 41,8 % en poids d'acrylamide pour le monomère à caractère non ionique
- 10,8 % en poids de monomère à charge cationique de même formule que celui de l'essai n° 2 à charge cationique,

et dont la viscosité spécifique est égale à 7,9.

Essai n° 22 :

Cet essai illustre l'invention et met en oeuvre un agent amphotère modificateur de rhéologie de phases lamellaires sous forme pulvérulente dont la composition monomérique est :

- 49,2 % en poids d'acide acrylique pour le monomère à charge anionique
- 40,0 % en poids d'acrylamide pour le monomère à caractère non ionique
- 10,8 % en poids de monomère à charge cationique de même formule que celui de l'essai n° 2 à charge cationique,

et dont la viscosité spécifique est supérieure à 10.

Essai n° 23 :

Cet essai illustre l'invention et met en oeuvre un agent amphotère modificateur de rhéologie de phases lamellaires sous forme pulvérulente dont la composition monomérique est :

- 29,3 % en poids d'acide acrylique pour le monomère à charge anionique
- 40,0 % en poids d'acrylamide pour le monomère à caractère non ionique
- 30,7 % en poids de monomère à charge cationique de même formule que celui de l'essai n° 2 à charge cationique,

et dont la viscosité spécifique est supérieure à 10.

Les résultats des mesures de viscosité Brookfield des divers essais sont rassemblés dans le tableau 3 suivant:

## TABLEAU 3

| | Essai n° | MODIFICATEUR DE RHEOLOGIE | | | | FORMULATION DETERGENTE | | | |
|---|---|---|---|---|---|---|---|---|---|
| | | Composition monomères | | | Viscosité Spécifique | Quantité (% en poids) | VISCOSITE BROOKFIELD ( mPa.s) | | |
| | | Anionique (% en poids) | Non ionique (% en poids) | Cationique (% en poids) | | | 10 T/min | 20 T/min | 100 T/min |
| TEMOIN | 20 | - | - | - | - | - | 3300 | 2100 | 1970 |
| INVENTION | 21 | 47,4 | 41,8 | 10,8 | 7,90 | 0,89 | 6200 | 3500 | 2000 |
| | 22 | 49,2 | 40,0 | 10,8 | > 10 | 0,89 | 30000 | 18750 | 5200 |
| | 23 | 29,3 | 40,0 | 30,7 | > 10 | 0,89 | 26000 | 16750 | 4900 |

La lecture du tableau 3 permet de constater l'épaississement c'est-à-dire l'augmentation de viscosité Brookfield à 10 tours/minute, 20 tours/minutes et 100 tours/minute de la composition détergente liquide anionique témoin, lorsqu'il

est fait usage, selon l'invention, d'un agent amphotère, modificateur de rhéologie de phases lamellaires, ayant une viscosité spécifique supérieure à 3 et préférentiellement supérieure à 10.

EXEMPLE 4 :

Cet exemple concerne l'épaississement d'une composition détergente liquide cationique par la mise en oeuvre selon l'invention, d'agent amphotère modificateur de rhéologie de phases lamellaires.

Dans ce but, pour chacun des essais de l'exemple on réalise avec le même mode opératoire et le même matériel que celui de l'exemple 2 la composition détergente liquide cationique composée de :

- 362,3 g d'eau
- 189 g de nonylphénol oxyéthylé 9 fois
- 31,5 g d'ammonium quaternaire commercialisé par la société CECA sous le nom NORAMIUM M2SH1 et
- 47,3 g de citrate trisodique
- Une fois les compositions réalisées, on ajoute de la même manière que dans l'exemple 2 et pour chaque essai 0,89 % en poids sec d'agent amphotère par rapport au poids total de la formulation avant de mesurer, au bout de 20 minutes d'agitation de la composition, les viscosités Brookfield 10 tours/minute, 20 tours/minute et 100 tours/minute à 25°C et à l'aide d'un viscosimètre Brookfield type RVT équipé du mobile adéquat.

Les différents essais effectués mettent en oeuvre les agents amphotères modificateurs de rhéologie de phases lamellaires suivants :

Essai n° 24 :

Cet essai est l'essai témoin dans lequel aucun agent amphotère n'est introduit.

Essai n° 25 :

Cet essai illustre l'invention et met en oeuvre le même agent amphotère modificateur de rhéologie de phases lamellaires que celui de l'essai n°21.

Essai n° 26 :

Cet essai illustre l'invention et met en oeuvre le même agent amphotère modificateur de rhéologie de phases lamellaires que celui de l'essai n°22.

Essai n° 27 :

Cet essai illustre l'invention et met en oeuvre le même agent amphotère modificateur de rhéologie de phases lamellaires que celui de l'essai n°23.

Les résultats des mesures de viscosité Brookfield des divers essais sont rassemblés dans le tableau 4 suivant :

# TABLEAU 4

| | Essai n° | MODIFICATEUR DE RHEOLOGIE | | | | FORMULATION DETERGENTE | | | |
|---|---|---|---|---|---|---|---|---|---|
| | | Composition monomères | | | Viscosité Spécifique | Quantité (% en poids) | VISCOSITE BROOKFIELD ( mPa.s) | | |
| | | Anionique (% en poids) | Non ionique (% en poids) | Cationique (% en poids) | | | 10 T/min | 20 T/min | 100 T/min |
| TEMOIN | 24 | - | - | - | - | - | 400 | 350 | 300 |
| INVENTION | 25 | 47,4 | 41,8 | 10,8 | 7,9 | 0,89 | 7200 | 4400 | 1750 |
| | 26 | 49,2 | 40,0 | 10,8 | > 10 | 0,89 | 16000 | 10500 | 3200 |
| | 27 | 29,3 | 40,0 | 30,7 | > 10 | 0,89 | 20000 | 12000 | 3600 |

La lecture du tableau 4 permet de constater l'épaississement c'est-à-dire l'augmentation de viscosité Brookfield

à 10 tours/minute, 20 tours/minute et 100 tours/minute de la composition détergente liquide cationique témoin, lorqu'il est fait usage, selon l'invention, d'un agent amphotère modificateur de rhéologie de phases lamellaires, ayant une viscosité spécifique supérieure à 3 et préférentiellement supérieure à 10.

EXEMPLE 5 :

Cet exemple concerne la fluidification de formulations anioniques cosmétiques de type shampooing, par l'utilisation selon l'invention d'agent amphotère modificateur de rhéologie de phases lamellaires en phase aqueuse.

Dans ce but, on teste l'activité fluidifiante d'un agent amphotère modificateur de rhéologie de phases lamellaires en phase aqueuse pour cinq formulations shampooing anioniques liquide différentes.

Essai n° 28 :

Cet essai est un témoin et concerne la fabrication d'un shampooing liquide dans lequel aucun agent amphotère n'est introduit.

Dans ce but, et avec le même mode opératoire et le même matériel que dans l'exemple 1, on prépare une composition de shampooing anionique liquide ayant les divers constituants suivants :

- 105,1 d'eau
- 0,8 g de soude perle
- 2,42 g de monoéthanolamine
- 24,2 g de lauryléthersulfate de sodium
- 70,2 g de nonylphénol oxyéthylé 9 fois
- 4,26 g de carbonate de soude
- 20 g de citrate trisodique

puis après 20 minutes d'agitation de la formulation shampooing ainsi réalisée, on mesure les viscosités Brookfield des différents essais à 10 tours/minute, 50 tours/minute et 100 tours/minute à 25°C et à l'aide d'un viscosimètre Brookfield type RVT équipé du mobile adéquat.

Essai n°29 :

Cet essai illustre l'invention et met en oeuvre le même agent amphotère modificateur de rhéologie de phases lamellaires que celui de l'essai n°9. Ainsi on rajoute, à la composition de l'essai n°28, une quantité de l'agent modificateur de rhéologie de phases lamellaires précité correspondant à 0,56 % en poids sec d'agent par rapport au poids total de la formulation.

Après 20 minutes d'agitation de la composition ainsi réalisée, on mesure les viscosités des différents essais à 10 tours/minute, 50 tours/minute et 100 tours/minute à 25°C et à l'aide d'un viscosimètre Brookfield type RVT équipé du mobile adéquat.

Essai n°30 :

On réalise une formulation shampooing liquide témoin avec les mêmes conditions opératoires et le même matériel que dans l'essai n°28 mais ayant la composition suivante :

- 105,1 g d'eau
- 0,8 g de soude perle
- 2,42 g de monoéthanolamine
- 34,2 g de lauryléthersulfate de sodium
- 60,2 g de nonylphénol oxyéthylé 9 fois
- 4,26 g de carbonate de soude
- 20 g de citrate trisodique

Essai n°31 :

Cet essai illustre l'invention et met en oeuvre le même agent amphotère modificateur de rhéologie de phases lamellaires que celui de l'essai n°9.

Ainsi on rajoute à la composition de l'essai n°30, une quantité de l'agent modificateur de rhéologie de phases

lamellaires précité correspondant à 0,56 % en poids sec d'agent par rapport au poids total de la formulation.

Après 20 minutes d'agitation de la composition ainsi réalisée, on mesure les viscosités des différents essais à 10 tours/minute, 50 tours/minute et 100 tours/minute à 25°C et à l'aide d'un viscosimètre Brookfield type RVT équipé du mobile adéquat.

Essai n°32 :

On réalise une troisième formulation shampooing liquide témoin avec les mêmes conditions opératoires et le même matériel que dans l'essai n°28 mais ayant la composition suivante :

- 100,0 g d'eau
- 0,8 g de soude perle
- 2,42 g de monoéthanolamine
- 24,2 g de lauryléthersulfate de sodium
- 70,2 g de nonylphénol oxyéthylé 9 fois
- 5,33 g de carbonate de soude
- 25 g de citrate trisodique

Essai n°33 :

Cet essai illustre l'invention et met en oeuvre le même agent amphotère modificateur de rhéologie de phases lamellaires que celui de l'essai n°9. Ainsi on rajoute à la composition de l'essai n°32, une quantité de l'agent modificateur de rhéologie de phases lamellaires précité correspondant à 0,56 % en poids sec d'agent par rapport au poids total de la formulation.

Après 20 minutes d'agitation de la composition ainsi réalisée, on mesure les viscosités des différents essai à 10 tours/minute, 50 tours/minute et 100 tours/minute à 25°C et à l'aide d'un viscosimètre Brookfield type RVT équipé du mobile adéquat.

Essai n°34 :

On réalise une quatrième formulation shampooing liquide témoin avec les mêmes conditions opératoires et le même matériel que dans l'essai n°28 mais ayant la composition suivante :

- 100,0 g d'eau
- 0,8 g de soude perle
- 2,42 g de monoéthanolamine
- 34,2 g de lauryléthersulfate de sodium
- 60,2 g de nonylphénol oxyéthylé 9 fois
- 5,33 g de carbonate de soude
- 25 g de citrate trisodique

Essai n°35 :

Cet essai illustre l'invention et met en oeuvre le même agent amphotère modificateur de rhéologie de phases lamellaires que celui de l'essai n°9. ainsi on rajoute à la composition de l'essai n°34, une quantité de l'agent modificateur de rhéologie de phases lamellaires précité correspondant à 0,56 % en poids sec d'agent par rapport au poids total de la formulation. Après 20 minutes d'agitation de la composition ainsi réalisée, on mesure les viscosités des différents essai à 10 tours/minute, 50 tours/minute et 100 tours/minute à 25°C et à l'aide d'un viscosimètre Brookfield type RVT équipé du mobile adéquat.

Essai n°36 :

On réalise une cinquième formulation shampooing liquide témoin avec les mêmes conditions opératoires et le même matériel que dans l'essai n°28 mais ayant la composition suivante :

- 100,0 g d'eau
- 0,8 g de soude perle
- 2,42 g de monoéthanolamine

- 44,2 g de lauryléthersulfate de sodium
- 50,2 g de nonylphénol oxyéthylé 9 fois
- 5,33 g de carbonate de soude
- 25 g de citrate trisodique

Essai n°37 :

Cet essai illustre l'invention et met en oeuvre le même agent amphotère modificateur de rhéologie de phases lamellaires que celui de l'essai n°9. Ainsi on rajoute à la composition de l'essai n°36, une quantité de l'agent modificateur de rhéologie de phases lamellaires précité correspondant à 0,56 % en poids sec d'agent par rapport au poids total de la formulation.

Après 20 minutes d'agitation de la composition ainsi réalisée, on mesure les viscosités des différents essais à 10 tours/minute, 50 tours/minute et 100 tours/minute à 25°C et à l'aide d'un viscosimètre Brookfield type RVT équipé du mobile adéquat.

Les résultats des mesures de viscosité Brookfield des divers essais sont rassemblés dans le tableau 5 suivant, sachant que pour tous les essais, les compositions obtenues selon l'invention, sont stables et homogènes.

## TABLEAU 5

| | Essai n° | MODIFICATEUR DE RHEOLOGIE: Compositions monomères: Anionique (% en poids) | Non ionique (% en poids) | Cationique (% en poids) | Viscosité Spécifique | FORMULATION COSMETIQUE: Quantité modificateur (% en poids) | VISCOSITE BROOKFIELD ( mPa.s): 10 T/min | 50T/min | 100 T/min |
|---|---|---|---|---|---|---|---|---|---|
| TEMOIN | 28 | - | - | - | - | - | 22000 | 2700 | 600 |
| INVENTION | 29 | 18,1 | 59,8 | 22,1 | 0,80 | 0,56 | 220 | 168 | 156 |
| TEMOIN | 30 | - | - | - | - | - | 2800 | 760 | 420 |
| INVENTION | 31 | 18,1 | 59,8 | 22,1 | 0,80 | 0,56 | 80 | 80 | 80 |
| TEMOIN | 32 | - | - | - | - | - | Mesure Impossible | 8000 | 5000 |
| INVENTION | 33 | 18,1 | 59,8 | 22,1 | 0,80 | 0,56 | 160 | 160 | 160 |
| TEMOIN | 34 | - | - | - | - | - | 53000 | 6900 | 1500 |
| INVENTION | 35 | 18,1 | 59,8 | 22,1 | 0,80 | 0,56 | 130 | 130 | 128 |
| TEMOIN | 36 | - | - | - | - | - | 1500 | 640 | 420 |
| INVENTION | 37 | 18,1 | 59,8 | 22,1 | 0,80 | 0,56 | 85 | 85 | 85 |

La lecture du tableau 5 permet de constater la fluidification c'est-à-dire la baisse de viscosité Brookfield à 10 tours/minute, 50 tours/minute et 100 tours/minute de la composition cosmétique liquide anionique témoin lorsqu'il est fait usage, selon l'invention d'un agent amphotère modificateur de rhéologie de phases lamellaires de viscosité spécifique comprise entre 0,3 et 3,0.

EXEMPLE 6 :

Cet exemple concerne l'épaississement de compositions cosmétiques par l'utilisation, selon l'invention, d'agent amphotère modificateur de rhéologie de phases lamellaires en phase aqueuse.

Dans ce but, on teste l'efficacité d'un agent amphotère modificateur de phases lamellaires en phase aqueuse pour une formulation shampooing liquide anionique et une formulation shampooing liquide cationique.

Essai n° 38 :

Cet essai est un essai témoin concernant la fabrication d'un shampooing liquide anionique sans ajout d'agent amphotère modificateur de rhéologie de phases lamellaires.

Dans ce but, et avec le même mode opératoire et le même matériel que dans l'exemple 1, on prépare une composition de shampooing anionique liquide ayant les divers constituants suivants :

- 325,2 g d'eau
- 1,6 g de soude perle
- 4,84 g de monoéthanolamine
- 68,4 g de lauryléthersulfate de sodium
- 120,4 g de nonylphénol oxyéthylé 9 fois
- 8,52 g de carbonate de soude
- 40 g de citrate trisodique

puis après 20 minutes d'agitation de la formulation shampooing ainsi réalisée, on mesure les viscosités Brookfield à 10 tours/minute, 20 tours/minute et 100 tours/minute à 25°C et à l'aide d'un viscosimètre Brookfield type RVT équipé du mobile adéquat.

Il est à remarquer qu'après un stockage au repos de quelques minutes, la formulation décante créant ainsi deux phases.

Essai n° 39 :

Cet essai illustre l'invention et met en oeuvre le même agent amphotère que celui de l'essai n° 22.

Ainsi on rajoute, à la composition de l'essai n°38, une quantité de l'agent modificateur de rhéologie de phases lamellaires précité correspondant à 0,89 % en poids sec d'agent par rapport au poids total de la formulation.

Après 20 minutes d'agitation de la composition ainsi réalisée, on mesure les viscosités Brookfield à 25°C à 10 tours/minute, 20 tours/minute et 100 tours/minute et à l'aide d'un viscosimètre Brookfield type RVT équipé du mobile adéquat.

Essai n° 40 :

Cet essai est un essai témoin concernant la fabrication d'une formulation shampooing liquide cationique sans ajout d'agent amphotère modificateur de rhéologie de phases lamellaires.

Dans ce but avec le même mode opératoire et le même matériel que dans l'exemple 2, on prépare une composition de shampooing cationique liquide par addition successive de :

- 201 g d'eau
- 105 g de nonylphénol oxyéthylé 9 fois,
- 17,5 g de NORAMIUM M2SH1
- 26,3 g de citrate trisodique

Puis après 20 minutes d'agitation, on mesure, à 25°C les viscosités des différents essais à 10 tours/minute, 20 tours/minute et 100 tours/minute à l'aide d'un viscosimètre Brookfield type RVT équipé du mobile adéquat.

Essai n° 41 :

35 Cet essai illustre l'invention et met en oeuvre le même agent amphotère modificateur de rhéologie de phases lamellaires que celui de l'essai n° 22.

Ainsi on rajoute, à la composition de l'essai n°40, une quantité de l'agent modificateur de rhéologie de phases lamellaires précité correspondant à 0,89 % en poids sec d'agent par rapport au poids total de la formulation puis après 20 minutes d'agitation, ou procède comme précédemment à la mesure des diverses viscosités Brookfield.

Les résultats des mesures de viscosité Brookfield des divers essais sont rassemblés dans le tableau 6 ci-après.

## TABLEAU 6

| | Essai n° | MODIFICATEUR DE RHEOLOGIE | | | | FORMULATION COSMETIQUE | | | |
|---|---|---|---|---|---|---|---|---|---|
| | | Compositions monomères | | | Viscosité Spécifique | Quantité (% en poids) | VISCOSITE BROOKFIELD ( mPa.s) | | |
| | | Anionique (% en poids) | Non ionique (% en poids) | Cationique (% en poids) | | | 10 T/min | 20 T/min | 100 T/min |
| TEMOIN | 38 | - | - | - | - | - | 900 | 500 | 180 |
| INVENTION | 39 | 49,2 | 40,0 | 10,8 | >10 | 0,89 | 3600 | 1400 | 740 |
| TEMOIN | 40 | - | - | - | - | - | 3800 | 2050 | 910 |
| INVENTION | 41 | 49,2 | 40,0 | 10,8 | >10 | 0,89 | 21200 | 12000 | 3480 |

La lecture du tableau 6 permet de constater l'épaississement c'est-à-dire l'augmentation de viscosité Brookfield

à 10 tours/minute, 20 tours/minute et 100 tours/minute des compositions shampooing liquide cationique ou anionique témoin, lorqu'il est fait usage, selon l'invention, d'un agent amphotère modificateur de rhéologie de phases lamellaires, ayant une viscosité spécifique supérieure à 3 et préférentiellement supérieure à 10.

EXEMPLE 7 :

Cet exemple concerne l'obtention de compositions détergentes liquides comprenant simultanément une phase lamellaire anionique et une phase lamellaire cationique ainsi que la compatibilité des phases obtenue grâce à l'utilisation d'agent amphotère modificateur de rhéologie de phases lamellaires en phase aqueuse.

Dans ce but, avec le même mode opératoire et avec le même matériel que dans l'exemple 1, on prépare une composition détergente liquide anionique ayant pour composants :

- 132 g d'eau
- 0,98 g de soude perle
- 2,42 g de monoéthanolamine
- 24,2 g de dodécylbenzène sulfonate de soude
- 65,45 g de nonylphénol oxyéthylé 9 fois
- 2,87 g de carbonate de soude
- 13,57 g de citrate trisodique

Parallèlement à cette formulation anionique, il est préparé, avec le même mode opératoire et le même appareillage que dans l'exemple 2, la composition cationique de l'exemple 2 qui pourrait correspondre à un adoucissant de linge utilisé en détergence.

Ces deux phases lamellaires d'anionicité opposée ayant été réalisées, on procède d'une part au simple mélange des deux phases dans différentes proportions avant d'effectuer dans les mêmes conditions opératoires et avec le même matériel que pour les essais précédents la mesure de viscosité Brookfield lorsque cela est possible, et d'autre part au mélange simultané des deux phases d'anionicité opposée dans les mêmes proportions que précédemment avec l'agent amphotère modificateur de rhéologie de phases lamellaires de l'essai n°9 en quantité équivalente à 0,56 % en poids sec d'agent par rapport au poids total du mélange avant d'en déterminer le comportement rhéologique par mesure des viscosités Brookfield 10 tours/minute, 50 tours/minute et 100 tours/minute selon le même mode opératoire et le même matériel que pour les essais précédents et après avoir observé la stabilité et l'homogénéité des mélanges obtenues selon l'invention.

Les différentes proportions testées sont pour l'essai n°42 :

- 84,4 % en poids de phase anionique
- 15,6 % en poids de phase cationique

pour l'essai n° 43 :

- 50 % en poids de phase anionique
- 50 % en poids de phase cationique

pour l'essai n° 44 :

- 15,7 % en poids de phase anionique
- 84,3 % en poids de phase cationique

Les résultats des mesures de viscosité Brookfield des divers essais sont rassemblés dans le tableau 7 suivant:

## TABLEAU 7

| Essai n° | | 42 | 43 | 44 |
|---|---|---|---|---|
| COMPOSITION | % anionique | 84,4 | 50 | 15,7 |
| | % cationique | 15,6 | 50 | 84,3 |
| VISCOSITE BROOKFIELD (mPa.s) | | | | |
| TEMOIN (0 % agent) | 10 T/mn | Mesure Impossible | 2400 | DECANTATION RAPIDE |
| | 50T/mn | Mesure Impossible | 760 | |
| | 100 T/mn | Mesure Impossible | 540 | |
| INVENTION (0,56 % agent) | 10 T/mn | 520 | 140 | 104 |
| | 50T/mn | 200 | 110 | 104 |
| | 100 T/mn | 160 | 110 | 104 |

La lecture du tableau 7 permet de constater que l'on obtient un mélange à la fois fluide c'est-à-dire présentant une faible viscosité Brookfield, stable et homogène c'est-à-dire sans décantation rapide lorsqu'il est fait usage, selon l'invention, d'un agent amphotère modificateur de rhéologie de phases lamellaires en phase aqueuse.

EXEMPLE 8 :

Cet exemple concerne l'obtention de compositions cosmétiques liquides comprenant simultanément une phase lamellaire anionique et une phase lamellaire cationique ainsi que la compatibilité des phases obtenue grâce à l'utilisation d'agent amphotère modificateur de rhéologie de phases lamellaires en phase aqueuse.

Dans ce but, avec le même mode opératoire et le même appareillage que dans l'essai n°38, on prépare, une composition shampooing liquide anionique de même composition que celle de l'essai n°38.

Parallèlement à cette formulation anionique, il est préparé, avec le même mode opératoire et le même appareillage que dans l'essai n°40, la composition cationique de l'essai n°40.

Ces deux phases lamellaires d'anionicité opposée ayant été réalisées, on procède d'une part au simple mélange des deux phases dans différentes proportions avant d'effectuer dans les mêmes conditions opératoires et avec le même matériel que pour les essais précédents la mesure de viscosité Brookfield à 10 tours/minute, 20 tours/minute et 100 tours/minute lorsque cela est possible, et d'autre part au mélange simultané des deux phases d'anionicité opposée dans les mêmes proportions que précédemment avec l'agent amphotère modificateur de rhéologie de phases lamellaires de l'essai o n° 22 en quantité équivalente à 0,89 % en poids sec d'agent par rapport au poids total du mélange avant d'en déterminer le comportement rhéologique par mesure des viscosités Brookfield selon le même mode opératoire et le même matériel que pour les essais précédents.

Les différentes proportions testées sont pour l'essai n°45 :

- 66,7 % en poids de phase anionique
- 33,3 % en poids de phase cationique

pour l'essai n° 46 :

- 50 % en poids de phase anionique
- 50 % en poids de phase cationique

pour l'essai n° 47 :

- 33,3 % en poids de phase anionique
- 66,7 % en poids de phase cationique

Les résultats des mesures de viscosité Brookfield des divers essais sont rassemblés dans le tableau 8 suivant :

## TABLEAU 8

| Essai n° | | | 45 | 46 | 47 |
|---|---|---|---|---|---|
| COMPOSITION | % anionique | | 66,7 | 50 | 33,3 |
| | % cationique | | 33,3 | 50 | 66,7 |
| VISCOSITE BROOKFIELD (mPa.s) | | | | | |
| TEMOIN (0 % agent) | | 10 T/mn | DECANTATION RAPIDE | DECANTATION RAPIDE | 56500 |
| | | 20 T/mn | DECANTATION RAPIDE | DECANTATION RAPIDE | 20000 |
| | | 100 T/mn | DECANTATION RAPIDE | DECANTATION RAPIDE | 2400 |
| INVENTION (0,89 % agent) | | 10 T/mn | 28000 | 22400 | 66000 |
| | | 20 T/mn | 17800 | 13000 | 39000 |
| | | 100 T/mn | 3200 | 4600 | 10800 |

La lecture du tableau 8 permet de constater que l'on obtient un mélange stable et homogène c'est-à-dire sans décantation rapide lorsqu'il est fait usage, selon l'invention,d'un agent amphotère modificateur de rhéologie de phases lamellaires en phase aqueuse.

EXEMPLE 9

Cet exemple concerne la détermination de la quantité minimale d'agent amphotère à ajouter dans les compositions détergentes ou cosmétiques pour modifier efficacement la rhéologie des phases lamellaires en phase aqueuse.

Dans ce but , à partir de la composition détergente liquide cationique témoin de l'essai n° 10, il est mis en oeuvre, avec le même mode opératoire et le même matériel que dans l'exemple 2, une quantité d'agent amphotère correspondant à 0,15% en poids sec de l'agent amphotère de l'essai n°9 par rapport au poids total de la formulation pour l'essai

n° 48 et correspondant à 0,45% en poids sec du même agent amphotère par rapport au poids total de la formulation pour l'essai n° 49.

De même à partir de la composition détergente liquide anionique témoin de l'essai n° 20, il est mis en oeuvre, avec le même mode opératoire et le même matériel que dans l'exemple 3, une quantité d'agent amphotère correspondant à 0,15% en poids sec de l'agent amphotère de l'essai n°22 par rapport au poids total de la formulaton pour l'essai n° 50 et correspondant à 0,30% en poids sec du même agent amphotère par rapport au poids total de la formulation pour l'essai n°51.

Les compositions des différents essais ainsi obtenues, les mesures des viscosités Brookfield sont effectuées dans les mêmes conditions et avec le même matériel que pour les essais précédents. Les résultats des diverses o mesures sont rassemblés dans le tableau 9 suivant:

## TABLEAU 9

| | Essai n° | MODIFICATEUR DE RHEOLOGIE | | | | FORMULATION DETERGENTE | | | |
|---|---|---|---|---|---|---|---|---|---|
| | | Compositions monomères | | | Viscosité Spécifique | Quantité modificateur (% en poids) | VISCOSITE BROOKFIELD ( mPa.s) | | |
| | | Anionique (% en poids) | Non ionique (% en poids) | Cationique (% en poids) | | | 10 T/min | 20T/min | 100 T/mi |
| TEMOIN | 10 | - | - | - | - | - | 14400 | 6700 | 1440 |
| | 48 | 18,1 | 59,8 | 22,1 | 0,80 | 0,15 | décantation | décantation | décantatio |
| INVENTION | 49 | 18,1 | 59,8 | 22,1 | 0,80 | 0,45 | 400 | 350 | 215 |
| TEMOIN | 20 | - | - | - | - | - | 3300 | 2100 | 1970 |
| | 50 | 49,2 | 40,0 | 10,8 | >10 | 0,15 | 9600 | 6700 | 1900 |
| INVENTION | 51 | 49,2 | 40 | 10,8 | >10 | 0,3 | 26000 | 9300 | 6150 |

La lecture du tableau 9 permet de constater que l'on obtient un mélange stable et homogène lorsqu'il est fait usage,

selon l'invention, d'au moins 0,25% en poids sec d'un agent amphotère modificateur de rhéologie de phases lamellaires en phase aqueuse par rapport au poids total de la composition.

## Revendications

1. Utilisation de copolymères amphotères comprenant au moins deux motifs monomériques dont le premier est composé d'un ou plusieurs monomères éthyléniques à charge anionique, le deuxième est composé d'un ou plusieurs monomères éthyléniques à charge cationique dont l'un au moins de ces monomères à charge cationique possède une structure tensio-active , et comprenant éventuellement un troisième motif qui est composé d'un ou plusieurs monomères éthyléniques à caractère non ionique pour modifier la rhéologie des phases lamellaires de compositions détergentes ou cosmétiques.

2. Utilisation de copolymères amphotères selon la revendication 1 caractérisée en ce que les compositions détergentes et cosmétiques sont liquides ou pâteuses.

3. Utilisation de copolymères amphotères selon l'une des revendications 1 ou 2 caractérisée en ce que les phases lamellaires de la composition détergente ou cosmétique sont anioniques ou cationiques ou sont un mélange de phases anioniques et cationiques.

4. Utilisation de copolymères amphotères selon l'une quelconque des revendications 1 à 3 caractérisée en ce que la modification de la rhéologie des phases lamellaires consiste en une fludification lorsque les compositions détergentes ou cosmétiques sont anioniques ou cationiques et lorsque le copolymère répond à la formule générale (I) :

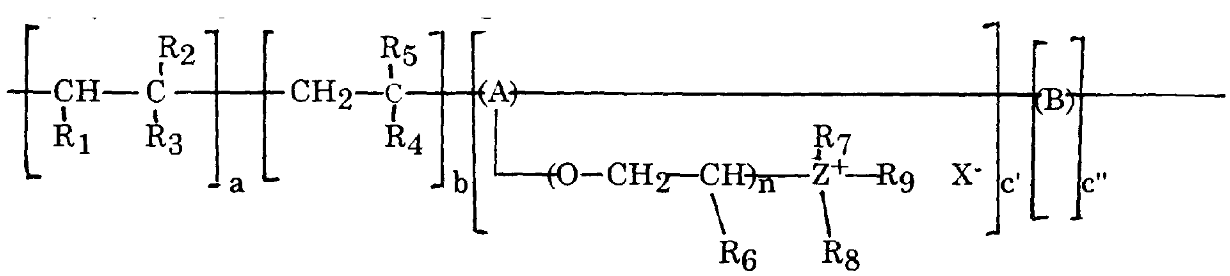

dans laquelle :

- Pour le motif de type anionique

  $R_1$ est H ou COOH
  $R_2$ est H ou $CH_3$
  $R_3$ est un groupement comportant au moins une fonction acide

  éventuellement totalement ou partiellement salifiée,

  et a représentant le pourcentage en poids par rapport à la masse totale en monomère, est compris, bornes incluses, entre 95 et 15.

- Pour le motif de type non ionique

  $R_4$ est
  $-CO-NH_2$, $-CO-OR_4'$, $-CO-NR_4''R_4'''$,

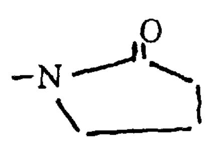

,

  dans lequel
  $R_4'$ est un radical alkyle ou oxyalkyle ayant 1 à 4 atomes de carbone,
  $R_4''$ est H ou un radical alkyle ayant 1 à 4 atomes de carbone,

$R_4'''$ est un radical alkyle ayant 1 à 4 atomes de carbone,
puis :
$R_5$ est H ou $CH_3$

et b représentant le pourcentage en poids par rapport à la masse totale en monomère, est compris, bornes incluses, entre 0 et 65. Pour le motif de type cationique,

- A représente le radical insaturé polymérisable et polymérisé, appartenant au groupe des esters acrylique, méthacrylique, maléique, itaconique, crotonique, vinylphtalique ainsi que les hémiesters maléique, itaconique, vinylphtalique ou encore les insaturés uréthannes tels que par exemple les acryluréthanne, méthacryluréthanne, α-α, diméthyl- m- isopropenylbenzyluréthanne, allyluréthanne ou bien encore les éthers allyliques, les acrylamide et méthacrylamide substituées ou non, des vinyliques.
- $R_6$ est H ou $CH_3$
- n = 2 à 30
- $R_7$ est un alkyle ayant 1 à 4 atomes de carbone
- Z est N ou S

X est un contre-ion sulfate ou halogènure et lorsque Z est N :

$R_8$ est une chaîne alkyle ayant 8 à 22 atomes de carbone ou un motif de formule :

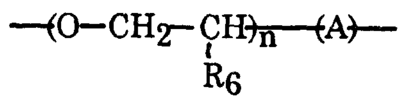

avec n = 2 à 30

et

$R_9$ est une chaîne alkyle ayant 8 à 22 atomes de carbone, et lorsque Z est S :

$R_8$ n'existe pas
$R_9$ est une chaîne alkyle ayant 8 à 22 atomes de carbone.

et c' représentant le pourcentage en poids, par rapport à la masse totale en monomère, du monomère à charge cationique possédant une structure tensio-active, est compris, bornes incluses, entre 5 et 60 et préférentiellement entre 10 et 35 avec c' + c" = c, c représentant le pourcentage en poids de tous les monomères cationiques et variant entre 5 et 60 et préférentiellement entre 10 et 35.

- B représente un motif monomérique cationique choisi parmi les monomères suivants : l'acrylate et/ou le méthacrylate de triméthyl amino éthyl chlorure et/ou sulfate, l'acrylamide et/ou le méthacrylamido de triméthylamino propyl chorure et/ou sulfate

et c" représentant le pourcentage en poids, par rapport à la charge totale en monomère, du monomère cationique ne possédant pas la structure tensio-active, est compris, bornes incluses, entre 0 et 55 avec c' + c" = c, c représentant le pourcentage en poids de tous les monomères à charge cationique et variant entre 5 et 60 et plus particulièrement entre 10 et 35. et lorsque le copolymère a une viscosité spécifique comprise entre 0,3 et 3,0 et préférentiellement entre 0,4 et 2,0.

**5.** Utilisation de copolymères amphotères selon l'une quelconque des revendications 1 à 3 caractérisée en ce que la modification de la rhéologie des phases lamellaires consiste en un épaississement lorsque les compositions détergentes ou cosmétiques sont anioniques ou cationiques et lorsque le copolymère répond à la formule générale I :

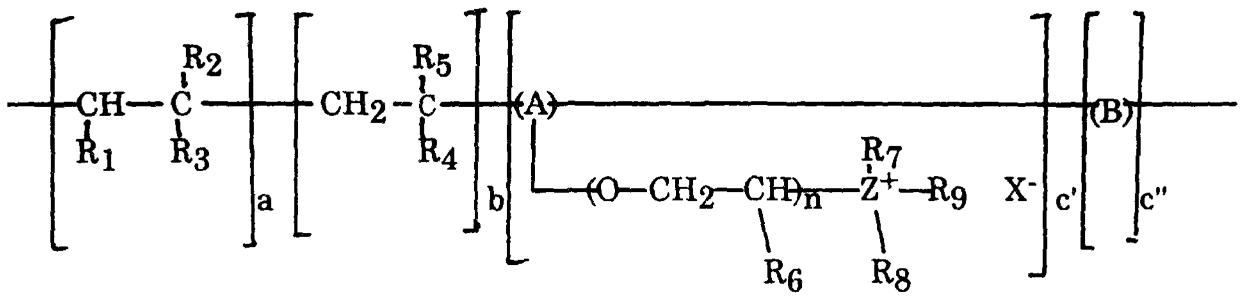

dans laquelle :

- Pour le motif de type anionique

  $R_1$ est H ou COOH
  $R_2$ est H ou $CH_3$
  $R_3$ est un groupement comportant au moins une fonction acide éventuellement totalement ou partiellement salifiée,

  et a représentant le pourcentage en poids par rapport à la masse totale en monomère, est compris, bornes incluses, entre 95 et 15.

  - Pour le motif de type non ionique

    $R_4$ est
    $-CO-NH_2$, $-CO-OR_4'$, $-CO-NR_4''R_4'''$,

    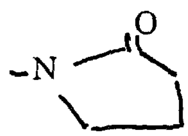
    ,

    dans lequel
    $R_4'$ est un radical alkyle ou oxyalkyle ayant 1 à 4 atomes de carbone,
    $R_4''$ est H ou un radical alkyle ayant 1 à 4 atomes de carbone,
    $R_4'''$ est un radical alkyle ayant 1 à 4 atomes de carbone,

    puis :
    $R_5$ est H ou $CH_3$
    et b représentant le pourcentage en poids par rapport à la masse totale en monomère, est compris, bornes incluses, entre 0 et 65.

Pour le motif de type cationique

- A représente le radical insaturé polymérisable et polymérisé, appartenant au groupe des esters acrylique, méthacrylique, maléique, itaconique, crotonique, vinylphtalique ainsi que les hémiesters maléique, itaconique, vinylphtalique ou encore les insaturés uréthannes tels que par exemple les acryluréthanne, méthacryluréthanne, α-α, diméthyl- m- isopropenylbenzyluréthanne, allyluréthanne ou bien encore les éthers allyliques, les acrylamide et méthacrylamide substituées ou non, des vinyliques.
- $R_6$ est H ou $CH_3$
- n= 2 à 30
- $R_7$ est un alkyle ayant 1 à 4 atomes de carbone
- Z est N ou S

  X est un contre-ion sulfate ou halogènure
  et lorsque Z est N :

    $R_8$ est une chaîne alkyle ayant 8 à 22 atomes de carbone ou un motif de formule :

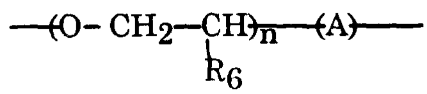

avec n = 2 à 30
et

$R_9$ est une chaîne alkyle ayant 8 à 22 atomes de carbone,
et lorsque Z est S :

$R_8$ n'existe pas
$R_9$ est une chaîne alkyle ayant 8 à 22 atomes de carbone. et c' représentant le pourcentage en poids, par rapport à la masse totale en monomère, du monomère à charge cationique possédant une structure tensio-active, est compris, bornes incluses, entre 5 et 60 et préférentiellement entre 10 et 35 avec c' + c" = c, c représentant le pourcentage en poids de tous les monomères cationiques et variant entre 5 et 60 et préférentiellement entre 10 et 35.

- B représente un motif monomérique cationique choisi parmi les monomères suivants : l'acrylate et/ou le méthacrylate de triméthyl amino éthyl chlorure et/ou sulfate, l'acrylamide et/ou le méthacrylamido de triméthylamino propyl chorure et/ou sulfate

et c" représentant le pourcentage en poids, par rapport à la charge totale en monomère, du monomère cationique ne possédant pas la structure tensio-active, est compris, bornes incluses, entre 0 et 55 avec c' + c" = c, c représentant le pourcentage en poids de tous les monomères à charge cationique et variant entre 5 et 60 et plus particulièrement entre 10 et 35.
et lorsque le copolymère a une viscosité spécifique supérieure à 3,0 et préférentiellement supérieur à 10.

**6.** Utilisation de copolymères amphotères selon l'une quelconque des 5 revendications 1 à 3 caractérisée en ce que la modification de la rhéologie des phases lamellaires consiste en une compatibilité des diverses phases lamellaires lorsque les compositions détergentes ou cosmétiques sont un mélange de phases anioniques et cationiques et lorsque le copolymère répond à la formule générale I :

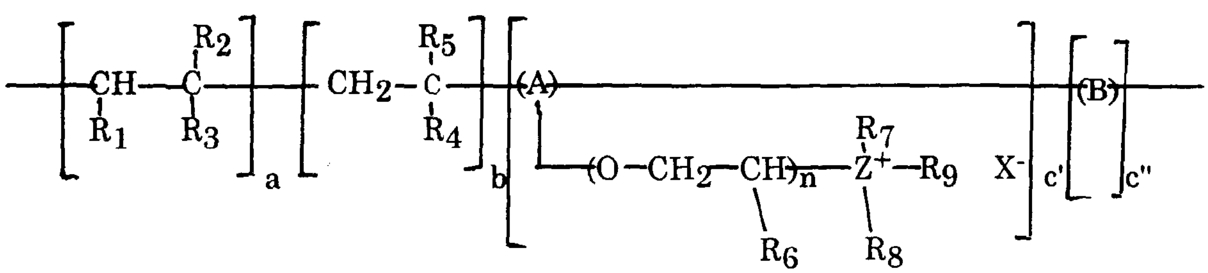

dans laquelle :

- Pour le motif de type anionique

$R_1$ est H ou COOH
$R_2$ est H ou $CH_3$
$R_3$ est un groupement comportant au moins une fonction acide éventuellement totalement ou partiellement salifiée,

et a représentant le pourcentage en poids par rapport à la masse totale en monomère, est compris, bornes incluses, entre 95 et 15.

- Pour le motif de type non ionique

$R_4$ est
$-CO-NH_2$, $-CO-OR_4'$, $-CO.NR_4''R_4'''$,

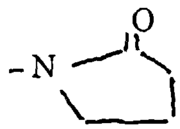

,

dans lequel

$R_4'$ est un radical alkyle ou oxyalkyle ayant 1 à 4 atomes de carbone,
$R_4''$ est H ou un radical alkyle ayant 1 à 4 atomes de carbone,
$R_4'''$ est un radical alkyle ayant 1 à 4 atomes de carbone,

puis :
$R_5$ est H ou $CH_3$
et b représentant le pourcentage en poids par rapport à la masse totale en monomère, est compris, bornes incluses, entre 0 et 65.

Pour le motif de type cationique

- A représente le radical insaturé polymérisable et polymérisé, appartenant au groupe des esters acrylique, méthacrylique, maléique, itaconique, crotonique, vinylphtalique ainsi que les hémiesters maléique, itaconique, vinylphtalique ou encore les insaturés uréthannes tels que par exemple les acryluréthanne, méthacryluréthanne, $\alpha$-$\alpha$, diméthyl- m- isopropenylbenzyluréthanne, allyluréthanne ou bien encore les éthers allyliques, les acrylamide et méthacrylamide substituées ou non, des vinyliques.
- $R_6$ est H ou $CH_3$
- n = 2 à 30
- $R_7$ est un alkyle ayant 1 à 4 atomes de carbone
- Z est N ou S X est un contre-ion sulfate ou halogénure
  et lorsque Z est N :

  $R_8$ est une chaîne alkyle ayant 8 à 22 atomes de carbone ou un motif de formule :

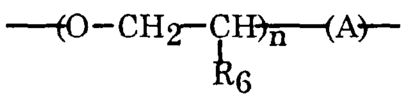

avec n = 2 à 30

et

$R_9$ est une chaîne alkyle ayant 8 à 22 atomes de carbone,

et lorsque Z est S :

$R_8$ n'existe pas
$R_9$ est une chaîne alkyle ayant 8 à 22 atomes de carbone.

et c' représentant le pourcentage en poids, par rapport à la masse totale en monomère, du monomère à charge cationique possédant une structure tensio-active, est compris, bornes incluses, entre 5 et 60 et préférentiellement entre 10 et 35 avec c' + c" = c, c représentant le pourcentage en poids de tous les monomères cationiques et variant entre 5 et 60 et préférentiellement entre 10 et 35.

- B représente un motif monomérique cationique choisi parmi les monomères suivants : l'acrylate et/ou le méthacrylate de triméthyl amino éthyl chlorure et/ou sulfate, l'acrylamide et/ou le méthacrylamido de triméthylamino propyl chorure et/ou sulfate et c" représentant le pourcentage en poids, par rapport à la charge totale en monomère, du monomère cationique ne possédant pas la structure tensio-active, est compris, bornes incluses, entre 0 et 55 avec c' + c" = c, c représentant le pourcentage en poids de tous les monomères à charge cationique et variant entre 5 et 60 et plus particulièrement entre 10 et 35.

**7.** Utilisation de copolymères amphotères selon l'une quelconque des revendications 1 à 6 caractérisée en ce que l'on utilise au moins 0,25% en poids sec dudit copolymère par rapport à la masse totale de la composition détergente ou cosmétique.

**8.** Agent de modification de rhéologie de phases lamellaires en phase aqueuse caractérisé en ce qu'il consiste en l'agent tel que défini dans l'une quelconque des revendications 1 à 7.

**9.** Composition détergente liquide contenant l'agent amphotère modificateur de rhéologie de phases lamellaires en phase aqueuse selon la revendication 8.

**10.** Composition détergente liquide selon la revendication 9 du type lessive liquide.

**11.** Composition cosmétique liquide ou pâteuse contenant l'agent amphotère modificateur de rhéologie de phases lamellaires en phase aqueuse selon la revendication 8.

**12.** Composition cosmétique selon la revendication 11 du type shampooing.

Office européen des brevets

# RAPPORT DE RECHERCHE EUROPEENNE

Numero de la demande
EP 96 42 0079

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | EP-A-0 458 599 (UNILEVER PLC. ) 27 Novembre 1991<br>* page 6, ligne 6 - page 9, ligne 26 *<br>* page 12, ligne 44 - page 16, ligne 25 *<br>* revendications 1,4 *<br>--- | 1-4,7-10 | C11D3/37<br>C11D17/00<br>A61K7/06<br>A61K7/00 |
| X | WO-A-91 06623 (UNILEVER PLC. ) 16 Mai 1991<br>* page 5, ligne 8 - page 10, ligne 29 *<br>* page 13, ligne 21 - page 15, ligne 34 *<br>* revendications 1-3,5 *<br>--- | 1-4,7-10 | |
| A | GB-A-2 104 091 (KAO SOAP CO. LTD. ) 2 Mars 1983<br>* le document en entier *<br>--- | 1-4,7-10 | |
| A | WO-A-92 17153 (HENKEL KGAA. ) 15 Octobre 1992<br>* page 6, ligne 5 - page 8, ligne 9 *<br>* exemples *<br>* revendications 1,6 *<br>--- | 1,4,7,8, 11,12 | **DOMAINES TECHNIQUES RECHERCHES (Int.Cl.6)** |
| D,A | US-A-5 275 809 (CHEN SHIH-RUEY T. ET AL.) 4 Janvier 1994<br>* revendications *<br>--- | 1,4,7,8, 11,12 | C11D<br>A61K |
| A | EP-A-0 522 756 (CALGON CORP. ) 13 Janvier 1993<br>* revendications *<br>--- | 1,4,7-12 | |
| D,A | EP-A-0 346 995 (UNILEVER NV. ) 20 Décembre 1989<br>* revendications 1-4 *<br>---<br>-/-- | 1,7-10 | |

Le présent rapport a été établi pour toutes les revendications

| Lieu de la recherche | Date d'achèvement de la recherche | Examinateur |
|---|---|---|
| LA HAYE | 7 Mai 1996 | Serbetsoglou, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numero de la demande
EP 96 42 0079

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | EP-A-0 112 592 (THE PROCTER & GAMBLE CO.) 4 Juillet 1984<br>* page 9, ligne 33 - page 11, ligne 11 *<br>* page 16, ligne 1 - page 17, ligne 29 *<br>* page 46, ligne 24 - page 49, ligne 20 *<br>* revendications 1-4,13,22,26 *<br>--- | 1-4,7-10 | |
| A | WO-A-92 04437 (HENKEL KGAA. ) 19 Mars 1992<br>* page 2, ligne 3 - page 3, ligne 14 *<br>* revendications 1,2 *<br>--- | 1-3,7-10 | |
| A | FR-A-2 470 596 (L' OREAL ) 12 Juin 1981<br>* page 2, ligne 1 - page 5, ligne 8 *<br>* page 19, ligne 17 - page 21, ligne 21 *<br>* page 23, ligne 3 - ligne 38 *<br>* revendications 1-4,12 *<br>----- | 1-4,7,8, 11,12 | |
| | | | DOMAINES TECHNIQUES RECHERCHES (Int.Cl.6) |

Le présent rapport a été établi pour toutes les revendications

| Lieu de la recherche | Date d'achèvement de la recherche | Examinateur |
|---|---|---|
| LA HAYE | 7 Mai 1996 | Serbetsoglou, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)